# EUROPEAN PATENT APPLICATION

(11) **EP 4 775 682 A1**
(43) Date of publication of application: **15.07.2026**
(21) Application number: 26150948.3
(22) Date of filing: 09.01.2026
(51) Int. Cl.: C12Q 1/6806

(54) **METHOD FOR OBTAINING THE SEQUECE OF IMMUNE RECEPTOR LIBRARIES FROM MAGNETICALLY ENRICHED CELLS BY ON-COLUMN CDNA SYNTHESIS**

(30) Priority: 10.01.2025 EP 25151084
(71) Applicant: Miltenyi Biotec B.V. & Co. KG, 51429 Bergisch Gladbach (DE)
(72) Inventor: HARDT, Olaf, 51429 Bergisch Gladbach (DE); EGOROV, Evgeny, 51429 Bergisch Gladbach (DE); SEREBROVSK, Ekaterina, 51429 Bergisch Gladbach (DE); CHUDAKOV, Dmitry, 51429 Bergisch Gladbach (DE)
(74) Representative: Kisters, Michael Marcus

(57) **Abstract**

The invention is directed to a method for determining the sequence of immune receptors expressed in target cells provided from a sample comprising target and non-target cells suspended in a fluid, especially TCR and BCR receptors.

## Description

The invention is directed to the generation of immune receptor libraries from magnetically enriched cells.

### BACKGROUND

Immune repertoire sequencing is a technology that is widely used in all areas of basic and clinical immunology research.

Immune receptor library generation is a multi-step process which includes isolation of a target cell population, cell lysis, RNA isolation, cDNA synthesis, and PCR amplification of target DNA fragments. On each of these steps, material loss can occur, leading to lower sensitivity and false-negative results.

Target immune cell populations are usually obtained via fluorescence-activated cell sorting, or through magnetic enrichment. Then, RNA isolation is carried out by one of commercial embodiments of phenol-chloroform extraction, or by a column-based approach. Then, cDNA is synthesized by a reverse transcription reaction with immune receptor specific primers. Finally, the immune receptor library is generated either by 5'RACE, or by a multiplex PCR-based approach, using primers specific to the immune receptor sequence.

RNA isolation is the main bottleneck of the process, when starting from a low number of cells. This is crucial when a researcher aims at isolating and generating immune receptor libraries from rare immune cell populations, such as T or B cells specific to a particular antigenic epitope.

### OBJECT OF THE INVENTION

The task of this invention is to provide a method which allows to combine target cell isolation, cell lysis and subsequent cDNA synthesis in one step, by performing the reverse transcription reaction directly on the magnetic column used for magnetic cell isolation.

Accordingly, object of the invention is a method for determining the sequence of immune receptors expressed in target cells provided from a sample comprising target and non-target cells suspended in a fluid **characterized in**
a) providing the sample with magnetic beads capable of specifically binding to the target cells
b) subjecting the thus obtained labelled cell sample to a magnetic field, thereby immobilizing the target cells and removing the non-target cells from the labelled sample
c) providing the immobilized target cells with a reverse transcriptase enzyme, one or more oligonucleotide primers and a detergent, wherein the detergent provides the reverse transcriptase enzyme and the oligonucleotide primers access to the mRNA coding for immune receptors and wherein the oligonucleotide primers specifically bind to mRNA coding for immune receptors, thereby coping the mRNA coding for immune receptors into c-DNA
d) multiplying the obtained cDNA by PCR into a library of cDNA
e) sequencing the library of cDNA, thereby obtaining the sequence of mRNA coding for immune receptors of the target cells
wherein step a), b) and c) are performed in the same reaction vessel.

### BRIEF DESCRIPTION OF THE DRAWINGS

Fig. 1 shows the assay principle and advantage over conventional RNA isolation-based approach. A - Cells are magnetically enriched on the column, and then RT reaction is carried out without eluting the cells, using the column as a reaction tube. B - Quantification of TCR libraries produced with the standard workflow (with RNA isolation), and with on-column cDNA synthesis approach.
Fig. 2 shows the overview of bioinformatic analysis workflow.
Fig. 3 shows the process overview for identification of influenza-specific TCRs by TCR profiling of MHC-multimer-enriched T cells ;
Fig. 4 shows the identification of influenza-specific TCRs by TCR profiling of MHC-multimer-enriched T cells. B - Flow cytometry characterization of intact PBMCs after MHC multimer staining. C - Flow cytometry characterization of enriched T cell fraction after MHC multimer staining and magnetic enrichment; D - Clonotypes identified as influenza-specific.
Fig 5 shows the identification of CEF-specific TCRs by TCR profiling of CD137-enriched T cells. A - workflow overview; B - Flow cytometry characterization of intact PBMCs after CD137 staining; C - Flow cytometry characterization of enriched T cell fraction after CD137 staining and magnetic enrichment.
Fig 6 shows identification of CEF-specific TCRs by TCR profiling of CD137-enriched T cells. D - Clonotypes identified as CEF-specific.
Fig. 7 shows the identification of a marker for T cell proliferating in vitro, and usage thereof for magnetic enrichment and antigen-specific TCR discovery. A - Identification of a marker specific for proliferating T cells - workflow overview; B - Flow cytometry characterization CD86-based magnetic enrichment;
Fig. 8 shows the identification of a marker for T cell proliferating in vitro, and usage thereof for magnetic enrichment and antigen-specific TCR discovery. C - Quantitative characteristics of an exemplary enrichment procedure. D - Clonotypes identified as SARS-COV-2_Spike-specific.
Fig. 9 shows the workflow overview for identification of CEF-specific TCRs by TCR profiling of CD137-enriched T cells.
Fig. 10 shows the identification of CEF-specific TCRs by TCR profiling of IFNγ-enriched T cells. B - Flow cytometry characterization of CEF-stimulated PBMCs after IFNγ staining; C - Flow cytometry characterization of nonstimulated PBMCs after IFNγ staining; D - Clonotypes identified as CEF- and SARS-COV-2_Spike-specific.
Fig. 11 A-D shows experimental data of of generating BCR libraries from B cells magnetically enriched from human PBMC samples for CD19 expression.

### DETAILED DESCRIPTION

In the method of the invention, the cells of the sample are provided with magnetic beads capable of specifically binding to the target cells The term "magnetic beads capable of specifically binding to the target cells" refers to any reagent/stain/label on the magnetic beads that allow for binding to the marker of interest on the surface of the target cells. The binding may be direct or indirect with the reagents as disclosed later.

The method of the invention preferable uses CD86 as a marker for the isolation of proliferating T cells.

After the staining, magnetically labelled cells are separated i.e. enriched on magnetic columns positioned in a magnetic field.

A reaction mixture containing a reverse transcriptase enzyme and a detergent for cell lysis was then added directly to the magnetic column. Then, the column was incubated at a working temperature for the reverse transcriptase enzyme. Afterwards, the products of reaction, containing cDNA of the target immune receptor, were eluted into conventional PCR tubes and purified by solid phase adsorption.

cDNA was then amplified by two subsequent PCR reactions with primers specific to certain regions of immune receptor gene sequences. The final product of the reaction, named immune receptor library, was subject to NGS sequencing and bioinformatic analysis.

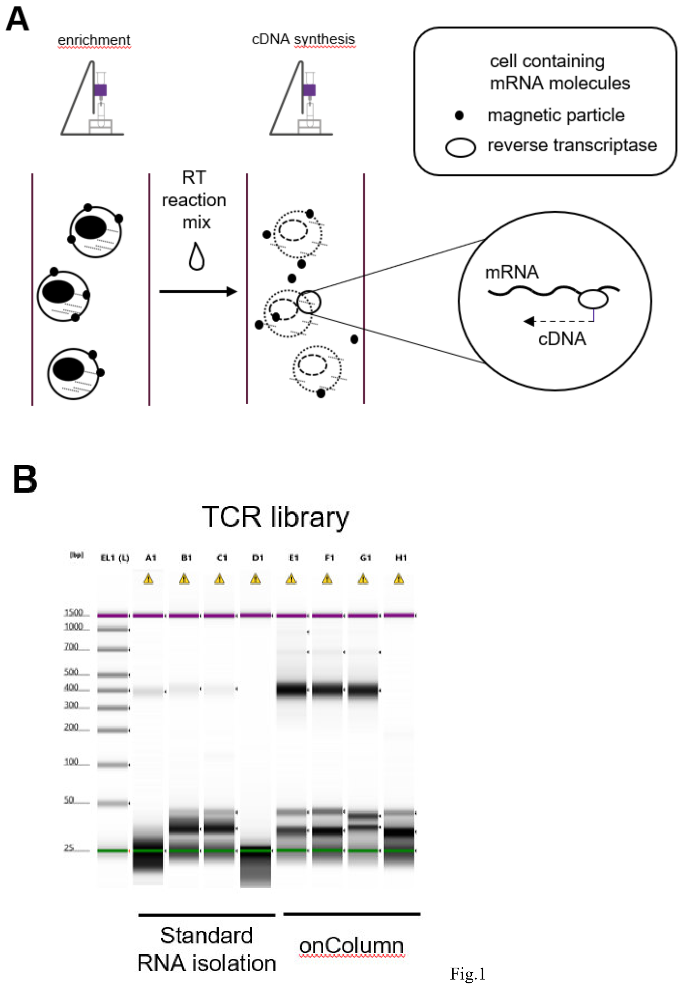

The principle of the method is represented on Fig.1 A.

The method comprises further steps to prepare cDNA libraries for NGS sequencing, where the starting material comprises intact cells bound to solid phase.

Starting material may comprise suspension of cells, either intact, or cultured, or stimulated.

Starting material may comprise cell suspension containing immune cells, either intact or stimulated with antigen and cultured for 24-240h.

After binding of the cells to the solid phase, the cDNA synthesis reaction is then carried out without eluting the cells from the solid phase matrix, with reaction mixture containing reverse transcriptase, primers, buffer and detergent.

The magnetic beads, the apparatuses to provide magnetic fields and the reaction vessel containing the paramagnetic matrix are known to the person skilled in the ant and/or commercially available.

Steps a) to c) and optionally step d) are performed in the same reaction vessel. Preferable, the reaction vessel comprises a paramagnetic matrix and that step a), b) and c) are performed in the paramagnetic matrix.

Further, steps a) to c) and optionally step d) are performed in the magnetic field.

The reaction vessel may be a tube-shaped vessel with an input opening at its upper end and an output opening at its lower end.

Optionally, the non-target cells and/or the cDNA is removed from the reaction vessel by eluding the fluid by gravitation through the output opening.

During step c), the output opening may be closed. In some situations, the fluids adhere to the paramagnetic matrix such that no fluids elude by gravitation alone. In such cases, the output opening may stay open during the whole method.

Preferable, the immune receptors are T cell or B cell receptors.

The paramagnetic matrix is also referred to as "Solid phase" and may comprise a ferromagnetic matrix within a nonmagnetic housing (column), and the cells may be labelled with paramagnetic particles through direct or indirect antibody*antigen and/or biotin*streptavidin interaction.

Solid phase may comprise a resin conjugated with a specific binder (an antibody, protein A, protein G, streptavidin), and the cells may be labelled with an unconjugated antibody, or an antibody, or multiple antibodies conjugated with biotin, PE, APC, or an MHC multimer conjugated with biotin, PE, APC, or an antigen-streptavidin tetramer).

Markers for enrichment may comprise cell surface molecules which may be recognized by specific interaction with another molecule. Markers used for cell enrichment may include CD137, CD154, CD86, CD98, CD279, CD25, CD38, CD69, IFNgamma, TNFa, TCR or BCR variable segment, TCR or BCR antigen-recognizing region.

Primers used for cDNA synthesis reaction may be barcoded (carry a unique nucleotide sequence that enables sample pooling and demultiplexing after NGS sequencing).

Primers used for cDNA synthesis may be a mix of oligonucleotides specific for the sequences of TCR alpha or beta chain constant domains.

Primers used for cDNA synthesis may be oligo-dT primers, to enable annealing to any mRNA for generation of total cDNA library.

Our approach allows to combine target cell isolation, cell lysis and subsequent cDNA synthesis in one step, by performing reverse transcription reaction directly on the magnetic column used for magnetic cell isolation.

The advantage of the described method over conventional approach is represented on Fig.1 B.

### direct labelling

In one embodiment, a specifically labelling reagent is selected from a protein, a protein fragment, a peptide, a polypeptide, a polypeptide fragment, an antibody, an antibody fragment, an antibody binding domain, an antigen, an antigen fragment, an antigenic determinant, an epitope, a hapten, an immunogen, an immunogen fragment, or any combination of any two or more thereof, coupled with a label. Label is then selected from a group of a protein, a protein fragment, a binding protein (BP), a binding protein fragment, an antibody, an antibody fragment, an antibody heavy chain, an antibody light chain, a single chain antibody, a single-domain antibody (a VHH for example), a Fab antibody fragment, an Fc antibody fragment, an Fv antibody fragment, a F(ab')2 antibody fragment, a Fab' antibody fragment, a single-chain Fv (scFv) antibody fragment, an antibody binding domain, an antigen, an antigenic determinant, an epitope, a hapten, an immunogen, an immunogen fragment, a binding domain; a metal ion, a metal ion-coated molecule; biotin, avidin, streptavidin; a substrate, an enzyme, an abzyme, a co-factor, a receptor, a receptor fragment, a receptor subunit, a receptor subunit fragment, a ligand, an inhibitor, a hormone, a binding site, a lectin, a polyhistidine, a coupling domain, an oligonucleotide, or a combination of any two or more thereof, coupled with a magnetic nanoparticle (direct labelling).

In a variant of the invention, the magnetic beads comprises a binding moiety for at least one antigen selected from the group consisting of CD4, CD7, CD8, CD19, CD20, CD3, CD45, CD45RA, CD45RO, CD137, CD154, CD86, CD98, CD279 (PD-1), CD274 (PD-L1), CD366 (TIM3), CD233 (LAG3), TIGIT, CD103, CD25, CD28, CD127, CD38, CD39, CTLA4, CD69, HLA-DR, CD40L, CD107a, CCR7, CXCR5, OX40, IFNgamma, TNFa, TCR or BCR constant or variable segment, TCR or BCR antigen-recognizing region, IgG, IgA, IgM, NKG2C, NKG2A, NKG2D, a chemokine receptor, or a KIR receptor.

### indirect labelling

In one embodiment, a specifically labelling reagent is selected from a protein, a protein fragment, a peptide, a polypeptide, a polypeptide fragment, an antibody, an antibody fragment, an antibody binding domain, an antigen, an antigen fragment, an antigenic determinant, an epitope, a hapten, an immunogen, an immunogen fragment, or any combination of any two or more thereof, coupled with a label. Label is then selected from a group of a protein, a protein fragment, a binding protein (BP), a binding protein fragment, an antibody, an antibody fragment, an antibody heavy chain, an antibody light chain, a single chain antibody, a single-domain antibody (a VHH for example), a Fab antibody fragment, an Fc antibody fragment, an Fv antibody fragment, a F(ab' )2 antibody fragment, a Fab' antibody fragment, a single-chain Fv (scFv) antibody fragment, an antibody binding domain, an antigen, an antigenic determinant, an epitope, a hapten, an immunogen, an immunogen fragment, a binding domain; a metal ion, a metal ion-coated molecule; biotin, avidin, streptavidin; a substrate, an enzyme, an abzyme, a co-factor, a receptor, a receptor fragment, a receptor subunit, a receptor subunit fragment, a ligand, an inhibitor, a hormone, a binding site, a lectin, a polyhistidine, a coupling domain, an oligonucleotide, or a combination of any two or more thereof.

Magnetic particle is provided in a second step, and comprises a binding moiety for a label described above, which is selected from a group of a protein, a protein fragment, a binding protein (BP), a binding protein fragment, an antibody, an antibody fragment, an antibody heavy chain, an antibody light chain, a single chain antibody, a single-domain antibody (a VHH for example), a Fab antibody fragment, an Fc antibody fragment, an Fv antibody fragment, a F(ab' )2 antibody fragment, a Fab' antibody fragment, a single-chain Fv (scFv) antibody fragment, an antibody binding domain, an antigen, an antigenic determinant, an epitope, a hapten, an immunogen, an immunogen fragment, a binding domain; a metal ion, a metal ion-coated molecule; biotin, avidin, streptavidin; a substrate, an enzyme, an abzyme, a co-factor, a receptor, a receptor fragment, a receptor subunit, a receptor subunit fragment, a ligand, an inhibitor, a hormone, a binding site, a lectin, a polyhistidine, a coupling domain, an oligonucleotide, or a combination of any two or more thereof (indirect labelling).

In a another variant of the invention, the magnetic beads comprise a binding moiety for a hapten or biotin and the target cells are provided with an antibody specific to one of antigens selected from the group consisting of CD4, CD7, CD8, CD19, CD20, CD3, CD45, CD45RA, CD45RO, CD137, CD154, CD86, CD98, CD279 (PD-1), CD274 (PD-L1), CD366 (TIM3), CD233 (LAG3), TIGIT, CD103, CD25, CD28, CD127, CD38, CD39, CTLA4, CD69, HLA-DR, CD40L, CD107a, CCR7, CXCR5, OX40, IFNgamma, TNFa, TCR or BCR constant or variable segment, TCR or BCR antigen-recognizing region, IgG, IgA, IgM, NKG2C, NKG2A, NKG2D, a chemokine receptor, or a KIR receptor wherein the antibody is labelled with a hapten or biotin

Haptens may include PE, APC, FITC, Cy5, Cy7 or biotin.

### Magnetic separation

Biological particles provided with magnetic nanoparticles via direct or indirect labelling are subjected to the magnetic field to separate the biological particles labelled with magnetic nanoparticles from the unlabeled biological particles.

In some embodiments, magnetic field is provided while the cell suspension is passing through the column. In other embodiments, magnetic field is provided when the cell suspension is in the tube.

After incubation in magnetic field, several washing steps may be performed, to eliminate non-target unlabeled cells. If enrichment is performed on the column, the washing solution is removed by gravity flow or by pressure. If enrichment is performed in the tube, the washing solution is removed by pipetting.

### cDNA synthesis

Reaction mixture for cDNA synthesis is provided in the same compartment where magnetic enrichment was performed. Target biological particles are not eluted from the column, are not removed from the tube, are not transferred to another vessel.

Reaction mixture contains, among other components, reverse transcriptase enzyme, deoxyribonucleotides, inorganic salts, detergent and cDNA synthesis primers.

The oligonucleotide primers may comprise a unique nucleotide sequence having 5 to 20 nucleotides as barcode.

In another variant, the oligonucleotide primers are capable of binding to the mRNA coding for TCR or BCR constant domains.

Preferable, the oligonucleotide primers are oligo-dT primers, capable of binding to all polyadenylated mRNA molecules.

cDNA synthesis primers are defined as a plurality of oligonucleotides which are capable of annealing to the target nucleic acid molecules in the conditions compatible with the reverse transcription reaction, and priming (initiating) said reaction.

The plurality of oligonucleotides comprises at least one barcode sequence (syBarcode), which is incorporated into the cDNA sequence and serves to identify cDNA molecules originating from one cDNA synthesis reaction.

Also, the plurality of oligonucleotides comprises a region capable of annealing to the target nucleic acid molecules.

In some embodiments, target nucleic acid molecules are mRNA molecules coding for TCRs or BCRs, and the plurality of oligonucleotides contains nucleotide sequence complementary to the TCR or BCR sequence.

In other embodiments, target nucleic acid molecules are all polyadenylated mRNA molecules, and the plurality of oligonucleotides contains oligo-dT sequence, which anneals to the polyA sequence of mRNA.

Once target bioparticles are provided with reaction mixture, reverse transcription reaction is carried out for 15-90 minutes at a temperature of 37 to 55 °C.

Techniques and conditions for reverse transcription reaction with template switching are well known in the art. Examples for protocols for reverse transcription with template switching can be found in Zhu et al, 2001 and Wellenreuther et al., 2004. Key elements are a specific polymerase and a template switching oligonucleotide. Commonly used polymerases are the Moloney Murine Leukemia Virus Reverse Transcriptase (MMLV-RT) and its derivates, or Thermostable group II intron reverse transcriptase (TIGRT).

Based on that target nucleic acids comprising the first oligonucleotide are generated, in other words barcoded target nucleic acids are generated.

### cDNA library amplification

Step 4 encompasses amplification of cDNA molecules by means of at least one PCR reaction. In some embodiments, two sequential PCR reactions are performed.

In some embodiments, a multiplex amplification reaction is used to amplify TCR or BCR cDNA. In some embodiments, a multiplex amplification reaction is used to amplify nucleic acid molecule(s) comprising at least a portion of a TCR or BCR CDR from cDNA derived from a biological sample. In some embodiments, a multiplex amplification reaction is used to amplify nucleic acid molecule(s) comprising at least two CDRs of a TCR or BCR from cDNA derived from a biological sample. In some embodiments, a multiplex amplification reaction is used to amplify nucleic acid molecules comprising at least three CDRs of a TCR or BCR from cDNA derived from a biological sample. In some embodiments, the resulting amplicons are used to determine the nucleotide sequences of the rearranged TCR or BCR CDRs in the sample.

In the multiplex amplification reactions, each primer set used target a same TCR or BCR region however the different primers in the set permit targeting the gene's different V(D)J gene rearrangements. For example, the primer set for amplification of rearranged TCR beta cDNA are all designed to target the same region(s) from the TCR beta gene but the individual primers in the set lead to amplification of the various rearranged TCR beta VDJ gene combinations. In some embodiments, at least one primer set includes a variety of primers directed to at least a portion of J gene segments of an immune receptor gene and the other primer set includes a variety of primers directed to at least a portion of V gene segments of the same gene.

### cDNA library sequencing

After amplification, there follows sequencing of the target nucleic acids, thereby obtaining sequencing data comprising sequences of the barcodes and the sequence of the target nucleic acid. Sequencing may be done according to methods known in the art. For example by sequencing by synthesis using one of the Illumina sequencing platforms (e.g. MiSeq, NextSeq or NovaSeq; Illumina, San Diego, CA, USA), by semiconductor sequencing using an Ion Torrent Next-Generation Sequencer, Thermo Fisher Scientific, Waltham, MA, USA), by long read sequencing using the PacBio Revio sequencing system (PacBio, Menlo Park, CA, USA), or by nanopore sequencing using one of the Oxford Nanopore sequencing systems (MinIon, GridION, PromethION; Oxford Nanopore, Oxford, United Kingdom).

In one embodiment of the invention additional sequencing adapters or barcodes may be attached to the target nucleic acids (e.g. as disclosed in
https://www.illumina.com/techniques/sequencing/ngs-library-prep/ligation.html.

### Data analysis

Step 6 is a data analysis step. Such a data analysis step may be done according to methods known in the art. In one embodiment of the invention, the sequencing data obtained in step 5 may be are aligned to sequence databases to determine the identity of the target nucleic acid. However the identity of the target nucleic acid may be assigned using alternative approaches. Identification of enriched immune receptor sequences is performed according to the methods known in the art. In one embodiment, bioinformatic analysis was based on EdgeR library, which allows for identification of overrepresented sequences in a set of samples relative to the other set of samples.

### Use of the invention

As evidenced by the examples, the method of the invention is useful to provide information on the following topics, for example for medical diagnostics:
- TCR profiling of T cells enriched for MHC multimer staining.
- TCR profiling of T cells enriched for CD137 upregulation.
- TCR profiling of T cells enriched for CD86 which is upregulated on proliferating cells.
- TCR profiling of T cells enriched for IFNγ secretion.
- BCR profiling of enriched B cells.
- Generation of total cDNA library from magnetically enriched cells.

### EXAMPLES

### General Method

Magnetic enrichment of target T cells was done using Multi-8 Molecular Columns (Miltenyi Biotec BV&Co KG, 130-092-444). PBMCs from a healthy donor were stained with the corresponding reagent (see Table 1), according to the manufacturers protocol, and then incubated with the anti-PE paramagnetic particles (Miltenyi Biotec, 130-048-801) or the anti-APC paramagnetic particles (Miltenyi Biotec,130-090-855). Molecular columns are placed on a suitable 96-well format magnet (for example, MultiMACS96 instrument from Miltenyi Biotec (130-091-937), then columns were primed with 70% ethanol, and subsequently equilibrated by separation buffer (PBS pH 7.2, 0.5% BSA and 2 mM EDTA), as recommended by manufacturer. Cells were washed and loaded on Molecular columns. After 2 times washing, RT mixture is loaded on the column. RT mixture contained a reverse transcriptase enzyme, appropriate buffer containing dNTPs, primers and detergent TRITON X-100 at 0.075% final concentration. Then, columns are incubated for 1h at a temperature of 37°C. Afterwards, RT mixture was eluted from the columns with mQ water into 0.2ml tube strips. The strips were incubated at 70°C for 10min in a thermocycler device to inactivate RT enzyme. Afterwards, the MiLaboratories kit for TCR profiling (THRM-001 or THRM-002) was used to generate TCR libraries for subsequent sequencing, according to the manufacturers protocol.

**Table 1. Experimental conditions**

| **E** | **Surface marker** | **Starting material** | **Conditions** | **Staining reagent** | **R** | **P** |
|---|---|---|---|---|---|---|
| 1 | Specific T cell receptor | PBMC, tumor infiltrating lymphocytes | IAV MP1, SARS-COV-2 S, control | MHC MACSimer A*0201 IAV MP1 (GILGFVFTL), human, PE MHC MACSimer A0201 SARS-COV-2 S (ZLQPRTFLL), human, PE; MHC MACSimer A0201control, human, PE , anti-PE microbeads | 4 | no |
| 2 | CD137 | PBMC | CEF PepTivator, no antigen | Anti CD137-PE antibody, anti-PE microbeads | 4 | 24 hou rs |
| 3 | CD86 | PBMC | CEF PepTivator, SARS-Cov-2_S PepTivator, no antigen | Anti CD86-APC antibody, anti-APC microbeads | 8 | 7 days |
| 4 | IFNgam ma surface capture | PBMC | CEF PepTivator, SARS-Cov-2_S PepTivator, no antigen | Interferon gamma capture kit, PE | 4 | 6 hou rs |
| 5 | CD19 | PBMC | unstimulated | Anti CD19-APC, anti-APC microbeads | | |
| 6, 7 | EpCAM (CD326) | BxPC-3, OVCAR-3 cell lines | unstimulated | Anti CD326-FITC antibody, anti-FITC microbeads | | |

| | | | | | | |
|---|---|---|---|---|---|---|
| E: No of Example R: Replicates per condition P: Pre-incubation with antigen | | | | | | |

### Bioinformatic pipeline

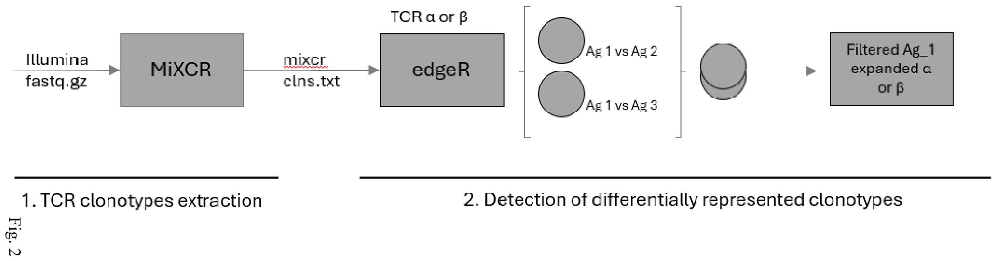

Bioinformatic pipeline for TCR Discovery NGS data analysis includes two main parts according to Fig. 2
1. Extraction of TCR-containing reads from raw fastq data and export of TCR clonotype tables (TCR repertoires) using MIXCR software.
2. Detection of differentially represented TCR clonotypes from bulk TCR repertoire

Detection of differentially represented (expanded/enriched) clonotypes was performed by comparing UMI frequencies of clonotypes in groups of samples using edgeR library from Bioconductor package. At least 4 replicates were analyzed per condition.

If detection of expanded/enriched clonotypes is not desired, it is possible also to perform immune receptor profiling of target cell populations without replicates.

### Example 1

Identification of Influenza MP1-specific TCR clonotypes by MHC multimer-based magnetic enrichment and subsequent TCR library generation with on-column cDNA synthesis.

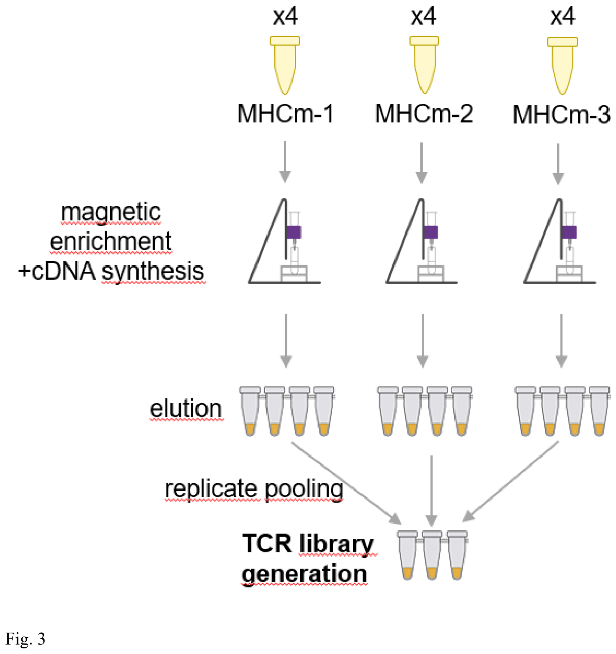

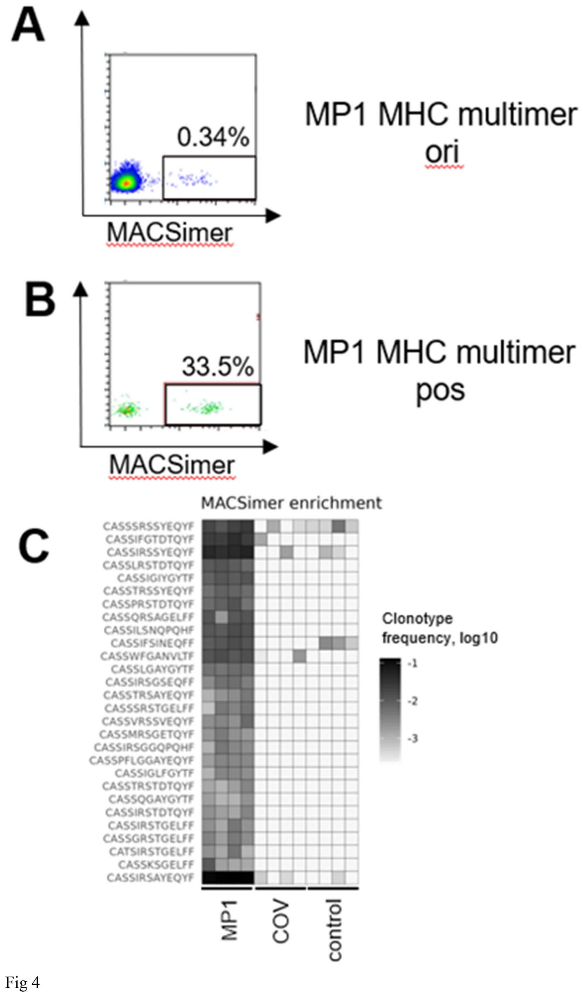

PBMCs isolated from blood of a healthy donor were stained with a MHC-multimer reagent, conjugated with PE, and then magnetically labelled with anti-PE-conjugated magnetic particles. Then, enrichment was performed using magnetic columns until the final wash step (without eluting cells from the columns). Next, reverse transcriptase reaction mixture was added to the columns, and the columns were incubated at 37°C for 1h to complete the cDNA synthesis reaction. Finally, product of the reaction was eluted from each column individually with mQ water, and then combined into one tube for cDNA purification and subsequent TCR library generation (Fig.3). In parallel, efficiency of the enrichment process was characterized by flow cytometry (Fig.4 A, B). TCR libraries were sequenced and analyzed using bioinformatic script designed to demultiplex pooled samples and identify enriched clonotypes (Fig.4 C). In a given example, 17 TCR clonotypes enriched with an MHC A*0201 multimer reagent specific for a GILGFVFTL epitope form influenza MP1 protein were identified.

### Example 2

Identification of TCR clonotypes reactive to the pool of mixed viral epitopes by CD137-based magnetic enrichment and subsequent TCR library generation with on-column cDNA synthesis.

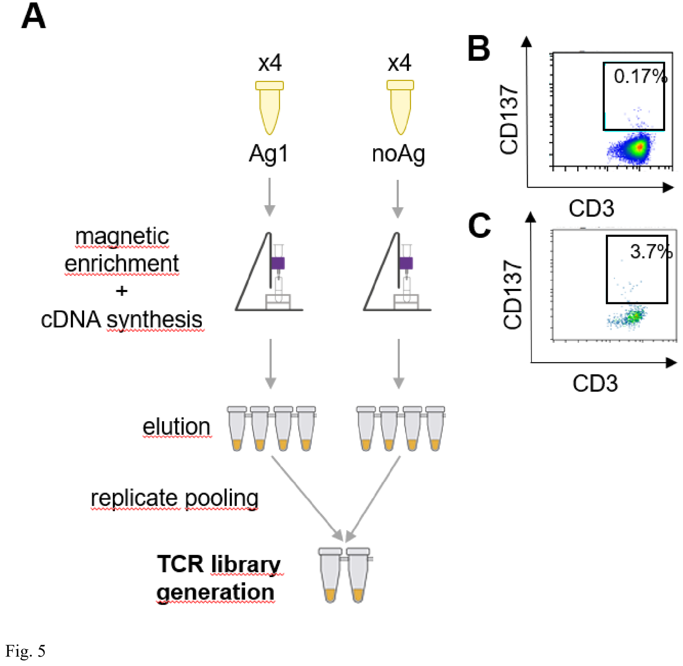

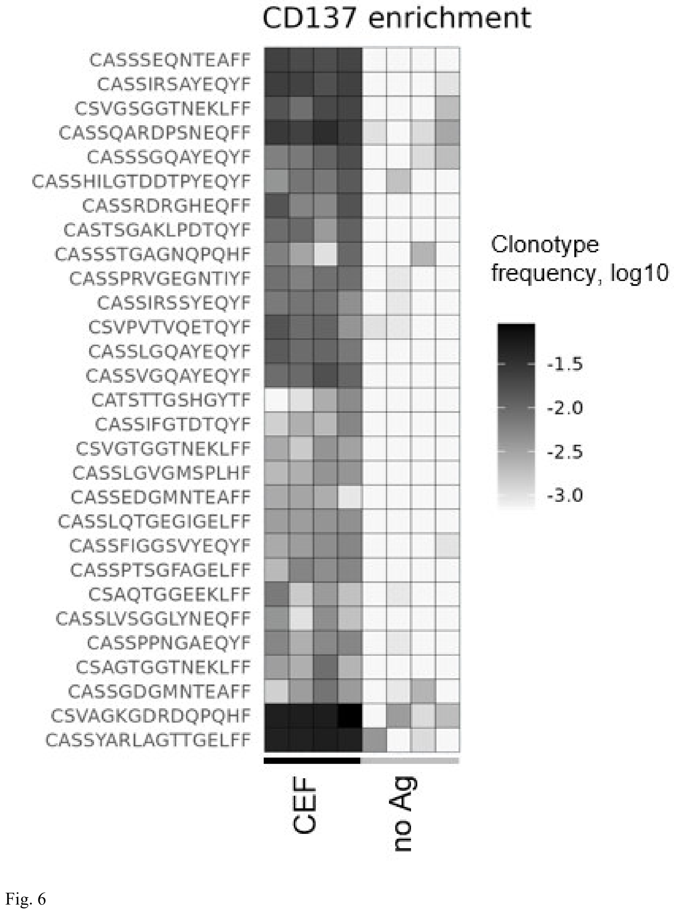

PBMCs isolated from blood of a healthy donor were stimulated with CEF peptide pool for 24h, and stained with a CD137 antibody, conjugated with PE, and then magnetically labelled with anti-PE-conjugated magnetic particles. Then, enrichment was performed using magnetic columns until the final wash step (without eluting cells from the columns). Next, reverse transcriptase reaction mixture was added to the columns, and the columns were incubated at 37°C for 1h to complete the cDNA synthesis reaction. Finally, product of the reaction was eluted from each column individually with mQ water, and then combined into one tube for cDNA purification and subsequent TCR library generation (Fig.5 A). In parallel, efficiency of the enrichment process was characterized by flow cytometry (Fig.5 B, C). TCR libraries were sequenced and analyzed using bioinformatic script designed to demultiplex pooled samples and identify enriched clonotypes (Fig.6). In a given example, 34 TCR clonotypes enriched with CD137 antibody after stimulation with CEF peptide pool were identified.

### Example 3

Identification of proliferating T cells after in vitro culturing of PBMCs with antigen.

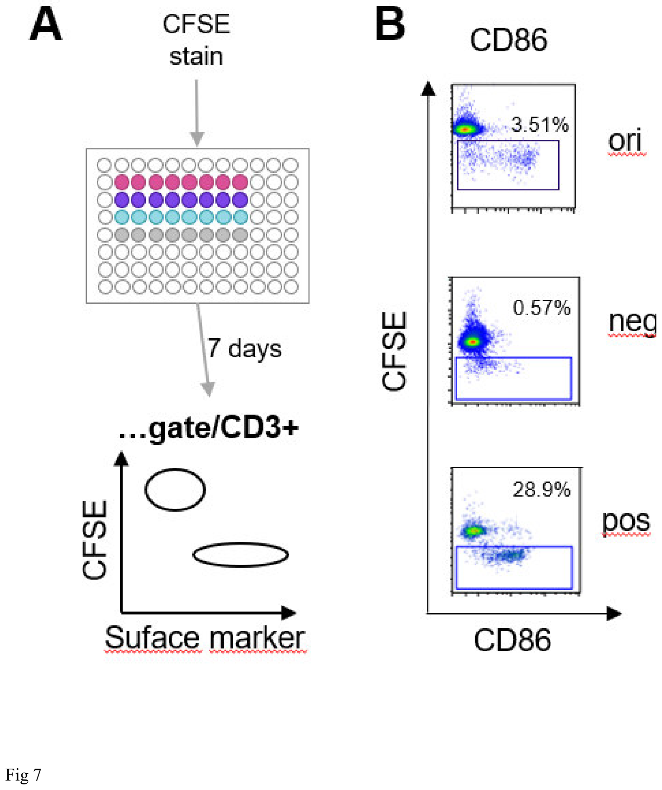

First, the screening for cell-surface markers correlating with a population of proliferating T cells after antigen-specific culture in vitro was carried out (Fig.7 A). Proliferating cells were identified by dye-dilution assay, where the cells are initially stained with a cell tracer dye, which then is diluted in the dividing cells to yield a population of cells with low fluorescence intensity compared to the non-dividing cells. After 7 d of antigen-specific proliferation, cells were stained individually with 386 antibodies towards various cell surface proteins. CD86 was identified as a marker that was specifically enriched in a population of dividing cells. In the following experiment, CD86 was used to enrich for the proliferating cells on magnetic columns, and subsequently generate TCR libraries with the on-column cDNA synthesis process (as described in examples 1 and 2).

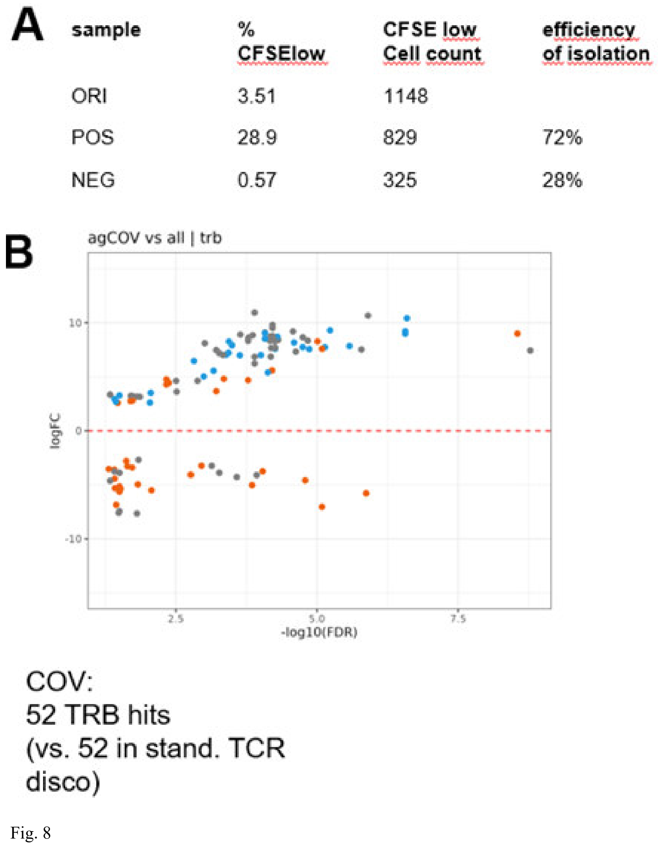

PBMCs isolated from blood of a healthy donor were stimulated with SARS-COV-2_Spike peptide pool and cultured for 7d, and stained with CD86 antibody, conjugated with APC, and magnetically labelled with anti-APC-conjugated magnetic particles. Then, enrichment was performed using magnetic columns until the final wash step (without eluting cells from the columns). Next, reverse transcriptase reaction mixture was added to the columns, and the columns were incubated at 37°C for 1h to complete the cDNA synthesis reaction. Finally, product of the reaction was eluted from each column individually with mQ water, and then combined into one tube for cDNA purification and subsequent TCR library generation. In parallel, efficiency of the enrichment process was characterized by flow cytometry (Fig.7 B, C). TCR libraries were sequenced and analyzed using bioinformatic script designed to demultiplex pooled samples and identify enriched clonotypes (Fig.8). In a given example, 52 TCR clonotypes enriched with CD86 antibody after stimulation with SARS-COV-2_Spike peptide pool were identified.

### Example 4

Identification of TCR clonotypes from the cells producing IFNγ in response to the antigen stimulation by magnetic enrichment and subsequent TCR library generation with on-column cDNA synthesis.

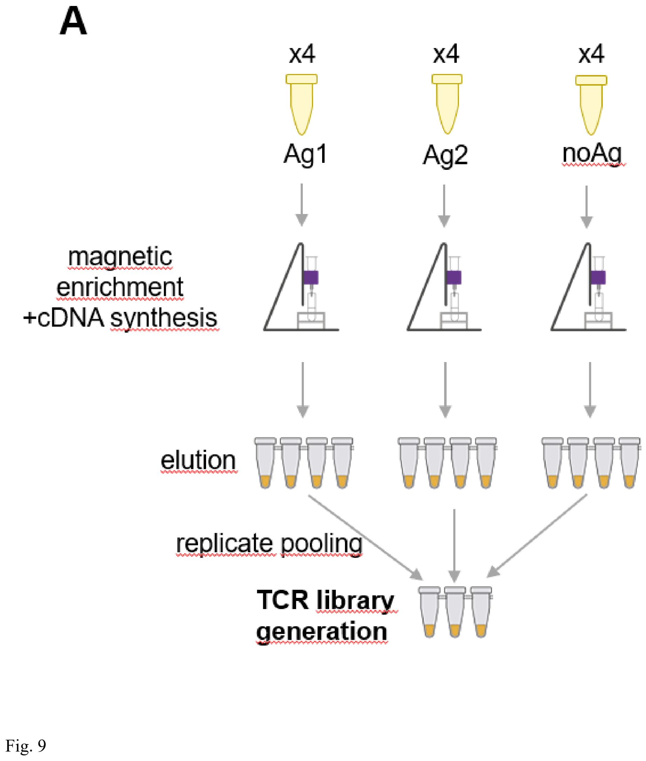

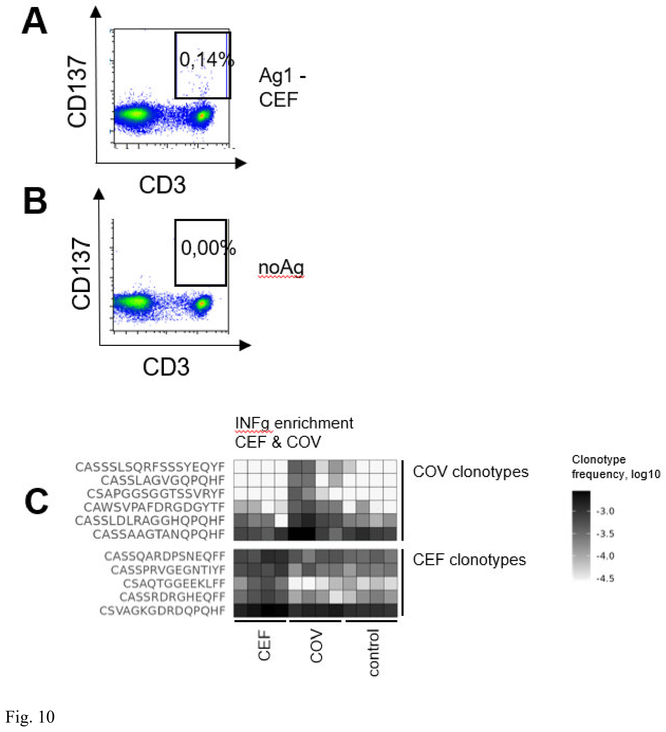

PBMCs isolated from blood of a healthy donor were stimulated with CEF and SARS-COV-2_Spike peptide pools for 6h, stained with an IFNγ-capture antibody, then with a PE-conjugated IFNγ-detection antibody, and lastly magnetically labelled with anti-PE-conjugated magnetic particles. Then, enrichment was performed using magnetic columns until the final wash step (without eluting cells from the columns). Next, reverse transcriptase reaction mixture was added to the columns, and the columns were incubated at 37°C for 1h to complete the cDNA synthesis reaction. Finally, product of the reaction was eluted from each column individually with mQ water, and then combined into one tube for cDNA purification and subsequent TCR library generation (Fig.9 A). In parallel, staining for IFNγ was checked with flow cytometry (Fig.10 A, B). TCR libraries were sequenced and analyzed using bioinformatic script designed to demultiplex pooled samples and identify enriched clonotypes (Fig.10 C). In a given example, 7 and 5 TCR clonotypes were identified as enriched after antigen stimulation and IFNγ capture protocol with SARS-COV-2_Spike and CEF, respectively.

### Example 5

Generation of BCR libraries from B cells magnetically enriched from human PBMC samples for CD19 expression.

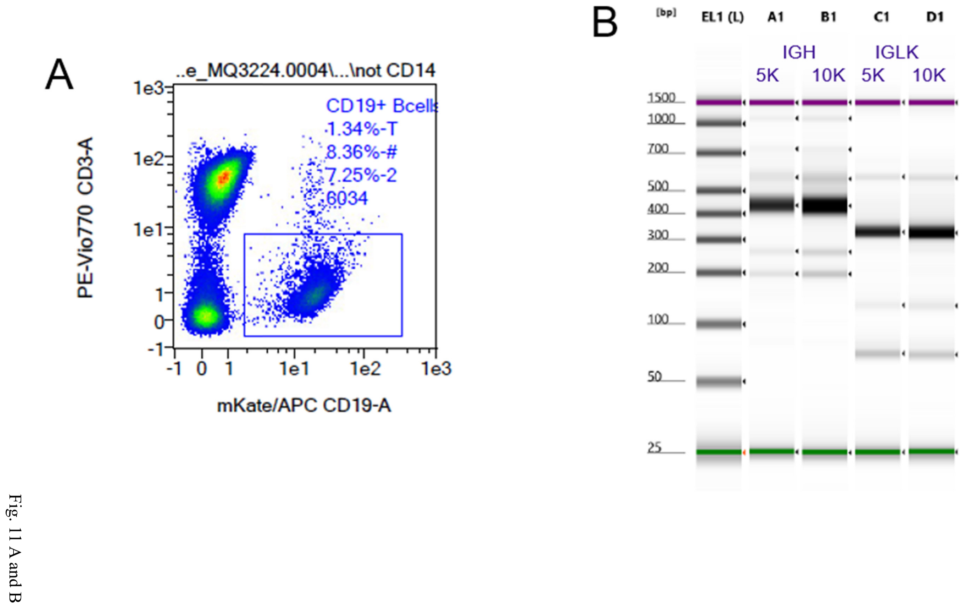

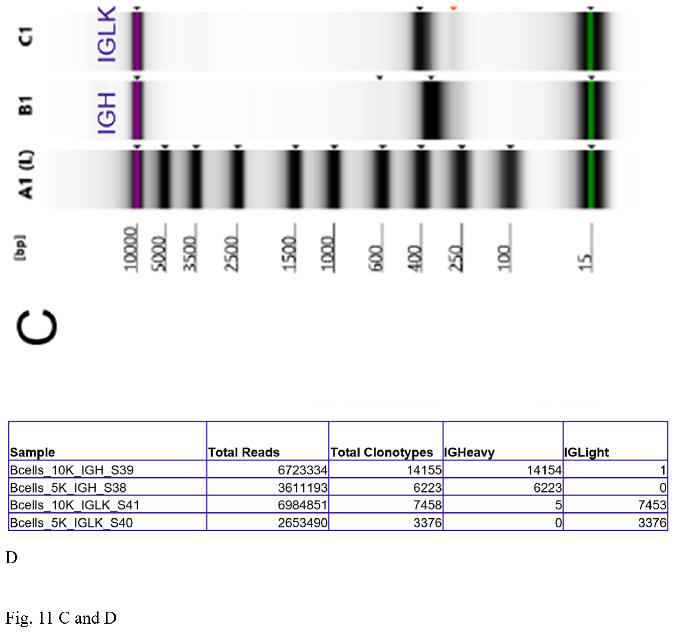

PBMCs isolated from blood of a healthy donor were stained with APC-conjugated anti CD19 antibody (Fig. 1 1 A), and then magnetically labelled with anti-APC-conjugated magnetic particles. Then, enrichment was performed using magnetic columns until the final wash step (without eluting cells from the columns). Next, reverse transcriptase reaction mixture was added to the columns, and the columns were incubated at 37°C for 1h to complete the cDNA synthesis reaction. Finally, product of the reaction was eluted from each column individually with mQ water, and then purified and subsequently used for generation of immunoglobulin heavy (IGH) and immunoglobulin light (IGL) chain libraries (Fig.11 B, C). BCR libraries were sequenced and analyzed using MIXCR software. In a given example, (Fig.11 D), 14155 IGH and 7458 IGL clonotypes were captured from a sample with calculated input of 10K B cells, and 6223 IGH and 3376 IGL clonotypes were captured from a sample with calculated input of 5K B cells.

### Example 6

Generation of total cDNA libraries from live mammalian cells magnetically labelled for EpCAM expression.

BxPC-3 and OVCAR-3 cells were stained with FITC-conjugated anti-CD326 antibody, and then magnetically labelled with anti-FITC-conjugated magnetic particles. Then, enrichment was performed using magnetic columns until the final wash step (without eluting cells from the columns). Next, reverse transcriptase reaction mixture containing oligo-dT primers with an adapter sequence, a template-switching oligo (TSO), and a detergent was added to the columns, and the columns were incubated at 42°C for 1h to complete the cDNA synthesis reaction. Finally, product of the reaction was eluted from each column individually with mQ water, purified with SPRI-select bead purification, and used in 5'RACE protocol. cDNA was amplified using a TSO-specific primer and adapter-specific primer, to produce an amplified cDNA library.

### Example 7

Generation of total cDNA libraries from live mammalian cells magnetically labelled for EpCAM expression.

BxPC-3 and OVCAR-3 cells were stained with FITC-conjugated anti-CD326 antibody, and then magnetically labelled with anti-FITC-conjugated magnetic particles. Then, enrichment was performed using magnetic columns until the final wash step (without eluting cells from the columns). Next, reverse transcriptase reaction mixture containing random hexamer primers with an adapter sequence, a template-switching oligo (TSO), and a detergent was added to the columns, and the columns were incubated at 42°C for 1h to complete the cDNA synthesis reaction. Finally, product of the reaction was eluted from each column individually with mQ water, purified with SPRI-select bead purification, and used in 5'RACE protocol. cDNA was amplified using a TSO-specific primer and adapter-specific primer, to produce an amplified cDNA library.

## Claims

1. Method for determining the sequence of immune receptors expressed in target cells provided from a sample comprising target and non-target cells suspended in a fluid **characterized in**
a) providing the sample with magnetic beads capable of specifically binding to the target cells
b) subjecting the thus obtained labelled cell sample to a magnetic field, thereby immobilizing the target cells and removing the non-target cells from the labelled sample
c) providing the immobilized target cells with a reverse transcriptase enzyme, one or more oligonucleotide primers and a detergent, wherein the detergent provides the reverse transcriptase enzyme and the oligonucleotide primers access to the mRNA coding for immune receptors and wherein the oligonucleotide primers specifically bind to mRNA coding for immune receptors, thereby coping the mRNA coding for immune receptors into c-DNA
d) multiplying the obtained cDNA by PCR into a library of cDNA
e) sequencing the library of cDNA, thereby obtaining the sequence of mRNA coding for immune receptors of the target cells
wherein step a), b) and c) are performed in the same reaction vessel.

2. Method according to claim 1 **characterized in that** the reaction vessel comprises a paramagnetic matrix and that step a), b) and c) are performed in the paramagnetic matrix.

3. Method according to claim 1 or 2 **characterized in that** step a), b) and c) are performed in the magnetic field.

4. Method according to any of claims 1 to 3, **characterized in that** the reaction vessel is a tube-shaped vessel with an input opening at its upper end and an output opening at its lower end.

5. Method according to claim 4 **characterized in that** removing the non-target cells and removing the cDNA is performed by eluding the fluid by gravitation through the output opening.

6. Method according to claims 4 or 5 **characterized in that** during step c), the output opening is closed.

7. Method according to any of claims 1 to 6 **characterized in that** the immune receptors are T cell or B cell receptors.

8. Method according to any of claims 1 to 7 **characterized in that** the magnetic beads comprise a binding moiety for at least one antigen selected from the group consisting of CD4, CD7, CD8, CD19, CD20, CD3, CD45, CD45RA, CD45RO, CD137, CD154, CD86, CD98, CD279 (PD-1), CD274 (PD-L1), CD366 (TIM3), CD233 (LAG3), TIGIT, CD103, CD25, CD28, CD127, CD38, CD39, CTLA4, CD69, HLA-DR, CD40L, CD107a, CCR7, CXCR5, OX40, IFNgamma, TNFa, TCR or BCR constant or variable segment, TCR or BCR antigen-recognizing region, IgG, IgA, IgM, NKG2C, NKG2A, NKG2D, a chemokine receptor, or a KIR receptor.

9. Method according to any of claims 1 to 7 **characterized in that** the magnetic beads comprise a binding moiety for a hapten or biotin and the target cells are provided with an antibody specific to one of antigens selected from the group consisting of CD4, CD7, CD8, CD19, CD20, CD3, CD45, CD45RA, CD45RO, CD137, CD154, CD86, CD98, CD279 (PD-1), CD274 (PD-L1), CD366 (TIM3), CD233 (LAG3), TIGIT, CD103, CD25, CD28, CD127, CD38, CD39, CTLA4, CD69, HLA-DR, CD40L, CD107a, CCR7, CXCR5, OX40, IFNgamma, TNFa, TCR or BCR constant or variable segment, TCR or BCR antigen-recognizing region, IgG, IgA, IgM, NKG2C, NKG2A, NKG2D, a chemokine receptor, or a KIR receptor wherein the antibody is labelled with a hapten or biotin

10. Method according to any of claims 1 to 9 **characterized in that** the oligonucleotide primers comprise a unique nucleotide sequence having 5 to 20 nucleotides as barcode.

11. Method according to any of claims 1 to 10 **characterized in that** the oligonucleotide primers are capable of binding to the mRNA coding for TCR or BCR constant domains.

12. Method according to any of claims 1 to 11 **characterized in that** the oligonucleotide primers are oligo-dT primers, capable of binding to all polyadenylated mRNA molecules.

13. Method according to any of claims 1 to 12 **characterized in that** the magnetic beads specifically binding to the target cells comprise a binding moiety for CD86.
